# EUROPEAN PATENT SPECIFICATION

(11) **EP 2 252 256 B1**
(45) Date of publication and mention of the grant of the patent: **01.08.2012**
(21) Application number: 09722798.7
(22) Date of filing: 19.03.2009
(51) Int. Cl.: A61K 8/81, A61K 8/891, A61Q 19/08

(54) **A COSMETIC TREATMENT METHOD INVOLVING PHOTO- POLYMERIZATION OF A COMPOSITION**
KOSMETISCHES BEHANDLUNGSVERFAHREN MIT PHOTOPOLYMERISIERUNG EINER ZUSAMMENSETZUNG
MÉTHODE DE TRAITEMENT COSMÉTIQUE COMPRENANT LA PHOTOPOLYMÉRISATION D'UNE COMPOSITION

(30) Priority: 20.03.2008 FR 0851823; 26.03.2008 US 64787 P
(43) Date of publication of application: 24.11.2010
(73) Proprietor: L'Oréal, 75008 Paris (FR)
(72) Inventor: SAMAIN, Henri, 91570 Bievres (FR); SIMONNET, Jean-Thierry, 94240 Cachan (FR)
(74) Representative: Tanty, François
(86) International application number: PCT/IB2009/051164
(87) International publication number: WO 2009/116006

(56) References cited:
- EP-A- 1 116 484
- EP-A- 1 527 770
- WO-A-01/70151
- WO-A-01/96450
- WO-A-2005/114329
- WO-A-2007/021707
- GB-A- 2 407 496
- US-A1- 2007 255 402
- OBARA Y ET AL: "External skin treatment agent for improvement of wrinkles comprises perfluoropolyether and water containing polymer" WPI / THOMSON,, 25 April 2000 (2000-04-25), XP002436694

## Description

The present invention relates to the treatment of keratinous materials and more particularly the skin, especially but not exclusively to smooth it in order to reduce the appearance of wrinkles, scars, or pores.

It is known to conceal skin blemishes using a covering composition applied to the skin. One difficulty linked with using such a composition is preserving a natural appearance.

There is a need for developing novel cosmetic treatment methods that can effectively reduce the visibility of skin blemishes or provide novel effects.

Exemplary embodiments of the invention provide a cosmetic treatment method comprising the steps consisting of:
a) depositing a composition on keratinous materials or on an artificial surface, for example a surface having relief that is based on that of keratinous materials, especially the skin, the composition being a polymerizable composition that can be polymerized under the action of a light stimulus;
b) directing a light beam onto the composition, especially a laser beam, to cause polymerization of the composition, especially biphotonic polymerization.

Biphotonic polymerization is synonymous with Multiphoton Absorption Polymerization (MAP). Optical nonlinearity of multiphoton absorption allows such absorption to be localized in regions of high light intensity. Phototransformation may be restricted to occur within the focal volume of a laser beam that has been focused through an optical system. By moving the focal position, three-dimensional microstructures may be created. Multiphoton absorption polymerization enables the creation of large-scale structures with high resolution features. A review of MAP is made in Multiphoton polymerization by Linjie Ji and John T. Fourkas in material today Volume 10, Issue 6, June 2007, pages 30-37, which is incorporated by reference.

The polymerization may be local.

The non-polymerized composition may be eliminated.

The beam may be moved relative to the keratinous materials.

The method may be carried out to modify the relief of the skin, in particular to smooth it.

The term "keratinous materials" means the skin, nails, lips, keratinous fibers, excluding the teeth. Exposure to a light stimulus may be carried out without interposing a mask between the source and the composition.

The term "polymerizable composition" means a composition comprising monomers or oligomers carrying one or more polymerizable functions, said polymerization possibly being radical polymerization or condensation polymerization.

For example, the polymerizable composition is a composition that cross-links under the action of a light stimulus, for example at ambient temperature.

In addition to the compound or compounds to be polymerized, the polymerizable composition may include at least one solvent, at least one polymerization initiator or other adjuvants such as at least one gelling agent or fillers, which may optionally be porous or active ingredients, especially anti-wrinkle or peeling agents, inter alia.

The polymerizable composition need not comprise polymerizable liquid crystals.

Before being polymerized, the composition may be fluid or gelled.

The light stimulus may be a visible or non-visible light stimulus, especially a UV or infrared light stimulus, and, for example, said stimulus may be light that is coherent or otherwise, in particular a laser beam, which may be continuous or pulsed.

The stimulus may be a monochromatic or polychromatic light stimulus comprising a dominant wavelength that can cause polymerization of the polymerizable material.

The light beam may be a beam of parallel, convergent, or divergent light. The light beam may be moved relative to the polymerizable composition, in particular as polymerization thereof proceeds.

The polymerizable composition may be deposited on the keratinous materials to be treated, in particular the skin, in order to cover recessed relief. The light beam may be directed onto this recessed relief to cause polymerization of the composition within said relief. By way of example, the relief may be selected from wrinkles, scars, and pores. The composition outside said relief may be left unexposed to light stimulus, so that it can be eliminated easily. The composition may initially extend beyond the recessed relief, with the non-polymerized composition being eliminated, or it may be deposited only in the recessed relief, avoiding such elimination.

For example, the polymerizable composition may be deposited to a thickness that may be from 0.2 µm [micrometer] to 2 mm [millimeter] and over an extent that is from 5 µm to 800 µm, for example.

Carrying out polymerization with a light beam brings with it many advantages.

Firstly, polymerization may occur relatively rapidly, for example over a few seconds, without requiring a supply of heat or the presence of a catalyst, which would otherwise be necessary with other polymerizable materials.

Polymerization may be carried out in situ precisely at the intended location, in particular within a wrinkle or a pore, using a light beam that is sufficiently narrow, especially a laser beam.

The light beam may be directed manually, with an operator monitoring the operation either with the naked eye or with an optical magnifying device, such as a binocular magnifier or a microscope, for example.

The light beam may be directed more or less automatically, for example as a function of a prior survey of the relief in the region to be treated.

The light beam may in particular be directed by a computer as a function of data contained in a topological database concerning the region to be treated. By way of example, said topological data may be acquired by any relief acquisition system, for example by projecting fringes or using a mobile camera or a plurality of cameras.

The light beam may be moved, for example pointed, manually or in a mechanized manner, for example using a device that can move an optical head from which the beam exits along at least two directions of a plane. The light beam may also be pointed by a control arm.

In a variant, regular or random scanning of the region to be treated is carried out and emission of the light beam is triggered only when the light beam can reach the zone to be polymerized. For example, the beam may always be pointed in the same direction, while being emitted during scanning only when the beam outlet window is locally superimposed on the zone to be treated.

Automatic tagging of the position of the beam hole relative to the zone to be polymerized may be carried out so that the device knows when the beam is to be emitted.

The light beam intended for polymerization may be moved along a path that corresponds to the polymerization to be carried out, either manually by the operator or automatically, for example. The operator may carry out a prior survey of the path, e.g. using a stylus that is moved over the zone to be treated, its movement being recorded, then the light beam being automatically directed along the same path.

Depending on the result that is desired, the polymerizable composition may have different mechanical properties after polymerization, in particular resulting in a material that is flexible or that is rigid.

A fairly rigid material may be preferred when treating pores and a flexible material may be preferred when treating wrinkles or scars.

Various parameters may be adjusted in order to regulate this rigidity: examples are the degree of polymerization, the photo-polymerizable material, in particular the choice of the chemical nature of the polymerizable materials, the spacers, and any side chains, the mixture of the various polymerizable materials, the presence of any cross-linking agents such as polyfunctional polymers or monomers, and/or adding other compounds such as organic or mineral materials, for example flexible or rigid particles, flexible or rigid polymers, solvents, or volatile or non-volatile oils, or plasticizers. Elastomers and adhesives are particularly preferred as added particles and polymers.

The polymerizable composition may be applied directly to the keratinous materials to be treated, especially the skin, or in a variant these may receive a prior preparation treatment.

The skin may in particular receive a base coat applied before depositing the polymerizable composition; as an example, the base coat may comprise a compound selected from adhesives or compounds that can be grafted, especially compounds that can be grafted onto proteins or compounds that may cause grafting or cross-linking functions to appear on the skin or on the photo-polymerizable material for application. Thus, according to exemplary implementations of the invention, the polymerizable composition is polymerized on skin that has been pre-treated with a reducing agent intended to cause free functions to appear. This can improve the hold of the material resulting from polymerization on the skin, e.g. in order to obtain adhesion over several hours, or even several days, where the composition has polymerized.

The polymerizable composition may be transparent or translucent, it may optionally be colored, and in particular it may incorporate at least one coloring agent, for example a dye or a pigment. The composition may initially be transparent or translucent and the material resulting from polymerization may be colored.

After the composition has polymerized, this coloring agent is trapped in the material, which contributes to its hold. Further, the polymerization process may permit at least one coloring agent to be introduced, for example into the polymerizable material, without destroying said coloring agent during the polymerization process. To this end, it is possible for the coloring agent to be selected from those with good resistance to light (in particular inorganic agents, but also certain dyes such as organic arylazo type dyes, or methine cyanines, or porphyrin or arylnitro dyes). It is also possible to select the energy parameters and the wavelength of the light beam. In particular, it is possible to select a wavelength that is different from the absorption wavelength of the coloring agent.

According to exemplary embodiments of the invention, the polymerizable composition includes at least one reactive coloring agent, for example a reactive dye or pigment, optionally along with at least one pre-dye or pro-pigment, capable of reacting either at the time of the polymerization process or afterwards, if the user so decides. The term "reactive dye or pigment" means, for example, dyes or pigments carrying thiol, masked thiol (for example thioesters), disulfide, dichlorotriazine, or acid chloride functions. Examples of pre-dyes are compounds such as those used to color the hair permanently: diaminobenzene and derivatives thereof, aminophenols and derivatives thereof, dihydroxybenzenes and derivatives thereof, and heterocycle derivatives

(amino or diamino or polyamino or hydroxy or dihydroxy or polyhydroxy, or aminohydroxy) such as indole, pyrazole, or pyridine derivatives. These agents are frequently colorless and when placed under the reaction conditions (presence of an oxidizing agent, for example) become colored and frequently insoluble.

The term "pro-pigment" means soluble forms that become insoluble after a reaction. Typically, one or more hydrosolubilizing groups is/are used (with hydroxy or amino or cationic or anionic functions), which are attached to the pro-pigments via cleavable functions (ester, for example). Under the action of an external stimulus, or naturally, the cleavable functions split off and the hydrosolubilizing groups are liberated from the pro-pigment, forcing the pigment to become insoluble.

Occasionally, the pro-pigment may become colored or may change color during the operation.

When carrying out the method, by estimating or observing the result, either visually or through a colorimetric detection apparatus, for example, the user may decide whether or not to tint the color of the material produced.

If it is decided to tint the color of the material produced, a reaction may be initiated using a stimulus adapted to cause the pre-dyes or pro-pigments to change.

As an example, it is possible to apply an oxidizing solution (for a coloring agent that is sensitive to oxidation) or an alkaline or acidic solution (for a coloring agent undergoing hydrolysis).

In one particular case, the coloring agents or other beneficial agents that are introduced carry a function that allows grafting to the polymerized substance. In particular, they may carry functions such as alcohols, acids, siloxanes, amines or phenols.

Another advantage of the invention is that it makes it possible to polymerize the polymerizable material while micro-structuring it relatively finely, for example with details of the order of 1 µm or less.

By way of example, micro-structuring may provide the material resulting from polymerization with a relief in the form of plateaus or of islands, or in the form of channels on the surface of the material. In a variant, or additionally, micro-structuring may also create pores especially when the polymerization is not carried out homogeneously through the entire thickness of the composition. The light beam may be focused inside the thickness of the layer of the composition in order to produce cavities.

For example, the micro-structuring may enable the micro-relief of the skin to be imitated.

The micro-structuring is preferably carried out by automatically controlling the light beam in accordance with a control program that is dependent on the micro-structure that is to be obtained or to be reproduced. In particular, the micro-structuring may be termed "3D", whereby it is possible to produce complex three-dimensional forms. Thus, one is not limited to "array" type micro-structures. As an example, it is possible to produce multi-layered materials in which each layer is separated from the next by a void, at least locally.

The micro-structuring may be precise and controlled. The thickness of the layers and the spacing between the layers may be controlled with a precision approaching or attaining 40 nm [nanometer] or less, for example.

The micro-structuring may in particular be used to modify the optical qualities of the material, for example by modulating its refractive index, its gloss or appearance, and/or to modify the mechanical properties of the material, for example by producing cavities that can not only increase the flexibility of the material and its capacity for deformation, but that can also modify the adhesion of the material, the presence of at least one cavity in the material allowing a resin, a binder or an adhesive to be inserted into the material.

The micro-structuring may also facilitate elimination of material, the presence of at least one cavity possibly facilitating its mechanical removal by allowing it to be detached using a small pair of tweezers, for example.

The micro-structuring may also improve the air permeability of the material after it has polymerized, and in particular it may prevent problems arising with occlusion of the skin.

By way of example, the micro-structuring may also create a diffraction pattern on the material; this diffraction pattern may produce a color by interference.

The pitch of such a pattern may be selected as a function of the color to be produced and, for example, as a function of the color of the treated keratinous materials. Typically, it may be of submicron order.

The thickness, color and/or micro-structure can produce different effects, for example in order to:
- conceal imperfections or re-create or improve the appearance of the skin, for example by imitating skin grain;
- reduce the visibility of the material on the skin.
   It may be almost invisible. Thus, a surface may be produced carrying small protuberances, e.g. directed perpendicularly to the surface for example (small dots of a few micrometers or small fibers). These protuberances provide a softening effect and/or can repel polluting agents and/or external agents such as water. They may also aid in eliminating perspiration and/or heat.

The material may also be intentionally visible. If so, it is intended to produce particular optical or visual effects, for example to produce a matt, a very glossy, or a colored appearance.

The micro-structuring may leave spaces for cell development, for example the development of skin cells or the development of added cells.

Preferably, the light beam is a pulsed laser beam obtained, for example, by using a flash pumped laser or diode pumped laser. By way of example, the pulse duration may lie in the range 10 femtoseconds to 1 ms [millisecond]. By way of example, the pulse frequency may be from 1 Hz [Hertz] to 100 kHz [kilohertz], preferably from 100 Hz to 25 kHz. By way of example, the energy per pulse may be from 0.01 µJ [microjoule] to 10 mJ [millijoule], preferably from 10 µJ to 2 mJ.

The energy density is high, preferably over short periods. Thus, a biphotonic polymerization reaction may be carried out.

In particular, a diode pumped laser may be used, termed a "femto", for example an "S PULSE" series laser from the supplier Amplitude, which has a characteristic wavelength of 1030 nm, or 515 nm after frequency doubling, a pulse duration of about 400 femtoseconds and a pulse energy of approximately 100 µJ. The pulse rate of the light beam may be 1000 Hz, for example.

If appropriate, the power of the laser may be reduced using at least one filter, a divergent lens and/or a diaphragm.

Examples of polymerizable materials that may be mentioned are those including a photo-reactive or photosensitive group, a chromophore, a dye or a pigment. Nonlimiting examples that may be mentioned are photosensitive organic/inorganic hybrid copolymers such as Ormocer.

As an example, polymerization is carried out by reacting vinyl functions, in different known forms and variants: acrylic, methacrylic, vinyl chloride. These functions are selected in order to have reactivity that is suitable for the illumination conditions and to the possible use of a catalyst and/or initiator. Thus, it is possible to use polymerizable materials carrying highly reactive functions such as cyanoacrylates or any anionic polymerization element.

As the main or as a complementary polymerizable compound, it is possible to use compounds that react by condensation, for example compounds including siloxane, alcohol, or isocyanate functions.

One polymerizable agent may carry different functions, for example a vinyl function and a function reacting by condensation.

It is possible to use combinations of polymerizable compounds, for example with different reactivities.

It is possible to use photo-cross-linkable compounds, possibly associated with acrylic compounds, epoxy compounds, or reactive silicones, for example those described in applications WO-01/96450 or GB-2 407 496.

By way of example, the light beam may be produced by using a device that may include fiber optics guiding a laser beam towards a system that is suitable for moving, in a controlled manner, means from which the light beam exits in at least two directions with high precision.

The device may allow the person or a part of the person to be treated to be immobilized during the treatment period.

As an example, the zone to be treated may be cooled.

During or before the treatment, other actions may be carried out, for example rubbing, applying aspiration or pressure, cleaning, and/or heating.

After polymerization, the non-polymerized material is eliminated, said elimination possibly being carried out by rinsing, aspiration, and/or wiping.

The polymerizable compound may be used for applications other than simply filling a wrinkle, a scar, or pores.

As an example, the polymerizable compound may be polymerized over a relatively extensive area, for example 20 cm² [square centimeter] or more, or even 100 cm² or more, in order to produce a second skin type coating, i.e. a continuous or substantially continuous coating that covers the skin to protect it or to modify its surface appearance, color, and/or gloss.

It may then be advantageous to micro-structure the coating. From a distance, it would appear continuous but on the microscopic scale it would have a variety of geometries in particular in order to imitate the micro-relief of the skin or to improve its respiration, to affect the retention of the material on the skin or to modify its appearance or gloss.

The material resulting from polymerization may be covered by a top coat, for example a make-up composition.

The invention may also find an application in treating the nails, the eyelashes, or the hair, as well as artificial surfaces.

In one example, an imprint of the skin is produced then a counter-print is produced using that imprint. Photo-polymerization is carried out on this counter-print and the material that is produced is applied to the skin.

By way of example, application may be performed as follows.

The counter-print may be produced in a material with a low surface tension, for example in a non-stick silicone substance.

It may also be produced from a normal substance that is covered with a thin film of a lubricating agent, for example a silicone oil.

The polymerizable composition is poured onto the counter-print and photo-polymerization is carried out in the counter-print.

Once polymerization is complete, an adhesive sheet is placed on the material resulting from polymerization, which sheet adheres to the material but not to the counter-print. Next, the material is removed and placed on the skin, which may have been pre-treated with an adhesive layer.

The sheet may be eliminated in various ways, for example by pulling gently or by rinsing. For this purpose, a hydrosoluble film may be selected, for example a cellulose derivative or a hydrosoluble resin or a hydrosoluble adhesive substance, for example a hydrosoluble resin.

According to exemplary embodiments of the invention, the sheet additionally contains agents that are beneficial for the skin, or beneficial for the polymerized material. By way of example, these may be particles modifying the mechanical properties of the material, such as silica or ceramic nanoparticles, polyamide particles, for example nylon, starch particles, polymeric polycaprolactone particles or nanoparticles, cellulose derivatives (for example cellulose acetobutyrate, cellulose acetopropionate, etc), polymers such as PVP (polyvinyl pyrrolidone), isophthalic derivatives such as AQ from Eastman Chemical, or polyurethanes, for example.

It is also possible to envisage introducing active ingredients that are released gradually from the material. Examples that may be mentioned are anti-wrinkle agents, sunscreens, moisturizers, agents reducing the visibility of pores, moisturizing agents, desquamating agents, agents improving the barrier function, de-pigmenting agents, anti-oxidants, anti-glycation agents, soothing and/or anti-irritant agents, sebo-regulators or anti-seborrheics, astringents, healing agents, anti-inflammatories, anti-acne agents, moisturizing agents: anti-glycation agents, NO-synthase inhibiters, agents for stimulating the synthesis of dermal or epidermal macromolecules and/or preventing their degradation, agents for stimulating the proliferation of fibroblasts and/or keratinocytes or for stimulating the differentiation of keratinocytes, other myorelaxation agents and/or dermo-relaxing agents, tightening agents, anti-pollution agents and/or radical scavengers, agents acting on the micro-circulation, agents acting on cell energy metabolism, and mixtures thereof.

Particular examples of agents that can reduce the visibility of pores that may be mentioned are:
- parakeratosis inhibitors, such as derivatives of amino acids and their salts, such as derivatives of glycine, aminodicarboxylic acids, acylaminodicarboxylic acids, pyrrolidinecarboxylic acids, piperidinecarboxylic acids, hexamethylene iminecarboxylic acid, beta-alanine and their salts and derivatives, for example those described in application EP-A-1 688 126 or WO-05/051340;
- halosalicylic acid derivatives such as those having at least one halogen substituent on the aromatic ring, for example those described in WO-05/058230;
- ascorbic acid, for example that described in DE-102004009150;
- a lotus extract, especially combined with methyl donors as described in WO-05/041996;
- a hair growth inhibitor, such as polyamine derivatives and/or analogs thereof, N-acetyl cysteine, neutralized salts of non-hydroxylated C₂-C₄₀ dicarboxylic acids, preferably malonate salts, for example those described in WO-05/120451;
- hydroalcoholic solutions, for example based on 1% to 60% of denatured ethanol, for example as described in WO-2006/056246; and
- mixtures thereof.

Examples of anti-wrinkle active ingredients that may be used in accordance with the invention are: retinol and its derivatives, such as retinyl palmitate; ascorbic acid and its derivatives, such as magnesium ascorbyl phosphate or ascorbyl glucoside; adenosine and its derivatives, in particular non-phosphated; tocopherol and its derivatives, such as tocopheryl acetate; nicotinic acid and its precursors such as nicotinamide; ubiquinone; glutathione and its precursors, such as L-2-oxothiazolidine-4-carboxylic acid; C-glycoside compounds and their derivatives, such as those described in particular below; plant extracts and in particular criste marine and olive leaf extracts, as well as plant proteins and their hydrolysates, such as rice or soya protein hydrolysates; extracts from algae, in particular laminaria; bacterial extracts; sapogenins such as diosgenin and Dioscorea extracts containing them, in particular wild yam extract; α-hydroxyacids; β-hydroxyacids such as salicylic acid or n-octanoyl-5-salicylic acid; oligopeptides and pseudopeptides and their acylated derivatives, in particular {2-[acetyl-(3-trifluoromethylphenyl)-amino]-3-methyl-butyrylamino} acetic acid and lipopeptides sold by the supplier SEDERMA under the trade names Matrixyl 500 and Matrixyl 3000; lycopene; manganese and magnesium salts, in particular gluconates; and mixtures thereof.

It is also possible to produce articles on a flat surface or in a solvent and to bond or apply these articles to the skin to obtain particular masking effects. To this end, a predetermined pattern is used for production.

It can imitate the appearance of skin grain. Once produced, the substance is applied or bonded to the skin. It is not very visible since its appearance approaches that of skin.

It may be almost invisible: thus, a surface may be produced having small protuberances (small dots of a few micrometers, or small fibers) in a perpendicular direction. These protuberances provide a soft effect and/or a polluting agent repellant effect, and/or can repel external agents such as water. They may also assist in eliminating perspiration or heat.

It may also be intentionally visible. If so, particular optical or visual effects may be created: as an example, it may provide a matt or very glossy or colored appearance.

In another example of an application, keratinous fibers, for example hair or eyelashes, are treated to insert them or to bond them to the body, in particular to produce extensions.

This may be carried out as follows:
- A flat layer of photo-polymerizable composition is deposited on a surface (the skin, the scalp, or some other surface).

Perpendicular to the surface, a series of keratinous fibers is deposited such that the ends of each fiber are in contact with the photo-polymerizable composition.

Polymerization is carried out. Polymerization is carried out in contact with one of the two ends of each fiber.

Polymerization may be carried out to create a micro-structure.

It is also possible to proceed as follows:
- Several small doses of photo-polymerizable composition are deposited flat on a surface (the skin, scalp or another surface). For example, each dose is a drop a few hundred micrometers to several millimeters in diameter. The doses may be separated.

Perpendicular to the surface, a series of keratinous fibers is deposited so that one end of each fiber is in contact with the photo-polymerizable composition. Each dose is in contact with one or more fibers.

Polymerization is carried out. Polymerization is carried out in contact with one of the two ends of each fiber.

The polymerization may be selected in order to create a micro-structure.

In a variant, the photo-polymerizable composition is applied to a suitable surface, in particular a non-planar surface, for example formed by cavities or carrying a mask formed from cavities. Each drop of polymerizable composition is placed in a cavity. This procedure can ensure that each drop does not flow to coalesce with its neighbors.

The fibers may be pre-treated to improve their retention in the material after photo-polymerization. For example, the pre-treatment may increase the area of contact between the fiber and the material.

Pre-treatment may comprise applying a spreading agent that allows the photo-polymerizable composition to climb up the fiber and hug it and/or may comprise producing micro-relief on the fiber (for example dots or hooks).

The pre-treatment may also allow better molecular bonding to be produced between the material and the fiber. To this end, the fiber may be treated to produce reactive functions, physical cross-linking functions and/or particular adhesion functions.

This is applicable to natural fibers and to artificial fibers. The articles that are bonded in this manner are not necessarily fibers. The articles may be spheres, polyhedra, or complex articles such as down.

The polymerizable composition may be deposited on the region to be treated using any means, in particular by spraying or using an applicator or a finger, for example a spatula, a foam, a pad, a brush, a capillary applicator, or a roll-on type applicator.

The polymerizable material may also be applied using a projection system, for example using an inkjet printer or by transfer, for example by applying a support on which the polymerizable material is present against the region to be treated.

The application may be carried out in a plurality of steps.

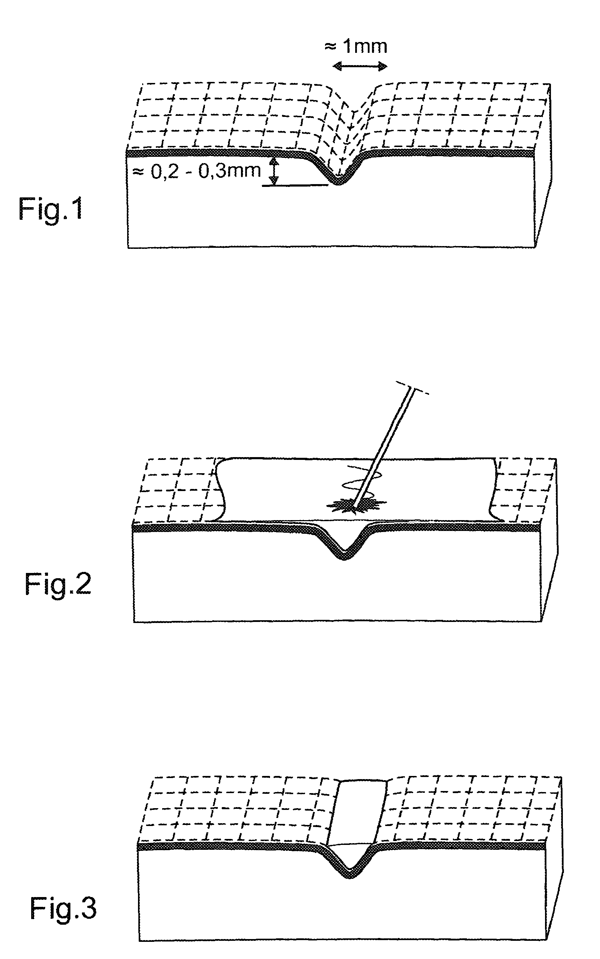

Figures 1 to 3 illustrate the application of the method to fill in a wrinkle.

In the step corresponding to Figure 1, the composition is applied. In the step corresponding to Figure 2, irradiation is carried out within the wrinkle. Next, the non-exposed composition is eliminated, as illustrated in Figure 3.

The invention also provides a system for carrying out photo-polymerization of a composition applied to human keratinous materials, in particular the skin, comprising:
- the composition that is polymerizable under the effect of a light stimulus;
- a device that can direct a light beam onto a deposit of a composition in order to cause its polymerization, especially a laser beam, for example with a view to producing biphotonic polymerization.

The invention also provides a material resulting from in situ photo-polymerization of a composition, on keratinous materials or on an artificial surface. This material may have a micro-structure reproducing the appearance of the keratinous materials, if appropriate.

The invention is not limited to the examples described above. The expression "comprising a" is synonymous with "comprising at least one".

## Claims

1. A cosmetic treatment method comprising:
a) depositing a composition on keratinous materials or on an artificial surface having relief that is based on that of keratinous materials, the composition being polymerizable under the action of a light stimulus;
b) directing a light beam from a laser onto the composition to cause biphotonic polymerization thereof.

2. A method according to claim 1, the polymerizable composition being deposited on the keratinous materials to be treated to cover recessed relief, the light beam being directed onto said recessed relief in order to cause polymerization of the material inside said relief.

3. A method according to claim 1 or 2, the light beam being directed manually, an operator monitoring the operation either with the naked eye or with an optical magnifying device.

4. A method according to claim 1 or 2, the light beam being directed automatically.

5. A method according to any one of claims 1 to 4, the keratinous materials receiving a base coat applied before depositing the polymerizable composition.

6. A method according to claim 5, wherein polymerization is carried out on skin that has been pre-treated with a reducing agent intended to cause free functions to appear.

7. A method according to any one of claims 1 to 6,
wherein the polymerizable composition incorporates at least one coloring agent.

8. A method according to any one of claims 1 to 7, the polymerizable composition including at least one dye or reactive pigment, and also optionally at least one pre-dye or pro-pigment, capable of reacting either at the time of the polymerization process or afterwards if the user so decides.

9. A method according to any preceding claim, wherein the composition is polymerized to micro-structure the material resulting from said polymerization.

10. A method according to claim 9, the micro-structuring being carried out using automatic control of the light beam, in accordance with a control program depending on the micro-structure that is to be obtained or reproduced.

11. A method according to any one of claims 1 to 10, the light beam being a pulsed laser beam the pulse duration being in the range 10 femtoseconds to 1 ms.

12. A method according to any preceding claim, elimination of the non-polymerized composition being carried out by rinsing, aspiration, and/or wiping.

## Patentansprüche

1. Kosmetisches Behandlungsverfahren, das umfasst:
a) Auftragen eines Stoffgemischs auf keratinöse Materialien oder auf eine künstliche Oberfläche, die Unebenheiten aufweist, die auf denen von keratinösen Materialien basieren, wobei das Stoffgemisch unter der Einwirkung eines Lichtreizes polymerisierbar ist;
b) Ausrichten eines Lichtstrahls von einem Laser auf das Stoffgemisch, um dessen biphotonische Polymerisation zu bewirken.

2. Verfahren nach Anspruch 1, wobei das polymerisierbare Stoffgemisch auf die keratinösen Materialien aufgetragen wird, die behandelt werden sollen, um vertiefte Unebenheiten abzudecken, wobei der Lichtstrahl auf die vertieften Unebenheiten ausgerichtet wird, um die Polymerisation des Materials innerhalb der Unebenheiten zu bewirken.

3. Verfahren nach Anspruch 1 oder 2, wobei der Lichtstrahl manuell ausgerichtet wird und eine Bedienperson die Einwirkung entweder mit bloßem Auge oder mit einer optischen Vergrößerungsvorrichtung überwacht.

4. Verfahren nach Anspruch 1 oder 2, wobei der Lichtstrahl automatisch ausgerichtet wird.

5. Verfahren nach einem der Ansprüche 1 bis 4, wobei die keratinösen Materialien eine Basisbeschichtung aufnehmen, die aufgebracht wird, bevor das polymerisierbare Stoffgemisch aufgetragen wird.

6. Verfahren nach Anspruch 5, wobei die Polymerisation auf Haut ausgeführt wird, die mit einem Reduktionsmittel vorbehandelt wurde, um freie Funktionen auftreten zu lassen.

7. Verfahren nach einem der Ansprüche 1 bis 6, wobei das polymerisierbare Stoffgemisch wenigstens ein Färbemittel enthält.

8. Verfahren nach einem der Ansprüche 1 bis 7, wobei das polymerisierbare Stoffgemisch wenigstens einen Farbstoff oder ein reaktives Pigment und außerdem optional wenigstens eine Farbvorstufe oder ein Pro-Pigment, die entweder zur Zeit des Polymerisationsprozesses oder später reagieren können, wenn der Anwender es bestimmt, umfasst.

9. Verfahren nach einem der vorhergehenden Ansprüche, wobei das Stoffgemisch polymerisiert wird, um für das Material, das aus der Polymerisation entsteht, eine Mikrostrukturierung auszuführen.

10. Verfahren nach Anspruch 9, wobei die Mikrostrukturierung unter Verwendung einer automatischen Steuerung des Lichtstrahls gemäß einem Steuerprogramm, das von der Mikrostruktur abhängt, die erhalten oder reproduziert werden soll ausgeführt wird.

11. Verfahren nach einem der Ansprüche 1 bis 10, wobei der Lichtstrahl ein gepulster Laserstrahl ist, wobei die Pulsdauer im Bereich von 10 Femtosekunden bis 1 ms liegt.

12. Verfahren nach einer der vorhergehenden Ansprüche, wobei die Entfernung des nicht polymerisierten Stoffgemischs durch Spülen, Absaugen und/oder Wischen ausgeführt wird.

## Revendications

1. Procédé de traitement cosmétique, comportant les étapes consistant à :
a) déposer sur les matières kératiniques ou sur une surface artificielle dont le relief est lié à celui des matières kératiniques, une composition polymérisable sous l'action d'un stimulus lumineux,
b) diriger sur la composition un faisceau lumineux issu d'un laser de façon à provoquer la polymérisation bi-photonique de celle-ci.

2. Procédé selon la revendication 1, la composition polymérisable étant déposée sur les matiéres kératiniques à traiter, de manière à recouvrir un relief en creux, le faisceau lumineux étant dirigé sur ce relief en creux de façon à provoquer la polymérisation du matériau à l'intérieur dudit relief.

3. Procédé selon l'une des revendications 1 et 2, le faisceau lumineux étant dirigé de manière manuelle, un opérateur contrôlant l'opération soit à l'oeil nu soit avec un dispositif d'agrandissement optique.

4. Procédé selon l'une des revendications 1 et 2, le faisceau lumineux étant dirigé automatiquement.

5. Procédé selon l'une quelconque des revendications 1 à 4, les matières kératiniques recevant une couche de base appliquée avant le dépôt de la composition polymérisable.

6. Procédé selon la revendication 5, dans lequel on réalise la polymérisation sur la peau que l'on aura traitée préalablement avec un réducteur destiné à faire apparaître des fonctions libres.

7. Procédé selon l'une quelconque des revendications 1 à 6, dans lequel la composition polymérisable incorpore au moins un agent de coloration.

8. Procédé selon l'une quelconque des revendications 1 à 7, la composition polymérisable comportant au moins un colorant ou pigment réactif, ainsi éventuellement qu'au moins un précolorant ou propigment, capable de réagir soit au moment du processus de polymérisation soit après, si l'utilisateur le décide.

9. Procédé selon l'une quelconque des revendications précédentes, dans lequel la composition est polymérisée de manière à microstructurer le matériau résultant de cette polymérisation.

10. Procédé selon la revendication 9, la microstructuration étant mise en oeuvre à l'aide d'un pilotage automatique du faisceau lumineux, selon un programme de commande dépendant de la microstructure que l'on cherche à obtenir ou à reproduire.

11. Procédé selon l'une quelconque des revendications 1 à 10, le faisceau lumineux étant un rayon laser pulsé, la durée d'impulsion étant comprise entre10 femtosecondes et 1 ms.

12. Procédé selon l'une quelconque des revendications précédentes, l'élimination de la composition on polymérisée s'effectuant par aspiration et/ou essuyage.
